# EUROPÄISCHE PATENTANMELDUNG

(11) **EP 0 080 105 A1**
(43) Veröffentlichungstag der Anmeldung: **01.06.1983**
(21) Anmeldenummer: 82110319.9
(22) Anmeldetag: 09.11.1982
(51) Int. Cl.: C07C 145/00, A01N 47/34

(54) **N-Sulfenylierte Biuret-N-carbonsäureester, ein Verfahren zu ihrer Herstellung sowie ihre Verwendung als Schädlingsbekämpfungsmittel**

(30) Priorität: 21.11.1981 DE 3146231
(71) Anmelder: BAYER AG, 51368 Leverkusen (DE)
(72) Erfinder: Kühle, Engelbert, Dr., D-5060 Bergisch Gladbach 2 (DE); Reinecke, Paul, Dr., D-5090 Leverkusen 3 (DE); Kuck, Karl-Heinz, Dr., D-4018 Langenfeld (DE)

(57) **Zusammenfassung**

Man erhält N-sulfenylierte Biuret-N"-carbonsäureester der Formel I, 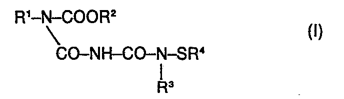 in welcher
R¹ und R² gleich oder verschieden sein können und für einen gegebenenfalls substituierten aliphatischen, cycloaliphatischen, aromatischen oder araliphatischen Rest stehen,
R³ für Wasserstoff oder für einen gegebenenfalls substituierten aliphatischen, cycloaliphatischen, araliphatischen oder aromatischen Rest steht und
R⁴ für einen Trihalogenmethylrest steht, durch Umsetzung von Acylisocyanat der Formel II 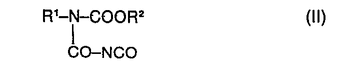 n welcher
R¹ und R² die oben angegebene Bedeutung haben, mit Sulfenamid der Formel 111, 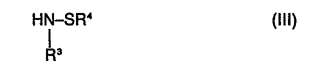 in welcher
R³ und R⁴ die oben angegebene Bedeutung haben, gegebenenfalls in Gegenwart eines Verdünnungsmittels.

Die Verbindungen der Formel I sind neu. Sie können als Schädlingsbekämpfungsmittel verwendet werden.

## Beschreibung

Die vorliegende Erfindung betrifft neue N-sulfenylierte Biuret-N"-carbonsäureester, ein Verfahren zu ihrer Herstellung und ihre Verwendung als Schädlingsbekämpfungsmittel, insbesondere als Fungizide und Bakterizide.

Seit langem sind Schwermetallsalze der Ethylen-1,2-bis- dithiocarbaminsäure in Landwirtschaft und Gartenbau zur Bekämpfung von pflanzenpathogenen Pilzen in Gebrauch (R. Wegler, Chemie der Pflanzenschutz- und Schädlingsbekämpfungsmittel, Band 2, S. 65 Springer Verlag Berlin, Heidelberg, New York 1970).

Weiterhin ist seit langem bekannt, daß N-Trihalogenmethylthiogruppen enthaltende Verbindungen als Fungizide in Landwirtschaft und Gartenbau verwendet werden können. So werden z.B. N-(Trichlormethylthio)-tetra- hydrophthalimid (DE-PS 887 506) und N,N-Dimethyl-N'- phenyl-N'-(fluordichlormethylthio)-sulfamid im Obst-und Weinbau zur Bekämpfung von Pilzkrankheiten praktisch eingesetzt (Angew. Chem. 76, 807 (1964)).

Weiterhin ist bekannt, daß die 2-[(2,3-Dichlorphenyl)-aminocarbony]-3,4,5,6-tetrachlorbenzoesäure (_{GB}-_{PS} 1 355 849) als Reisbakterizid eingesetzt werden kann.

Die Wirkung der genannten Verbindungen ist jedoch insbesondere bei niedrigen Aufwandmengen und -konzentrationen nicht immer ganz befriedigend.

Es wurden neue N-sulfenylierte Biuret-N"-carbonsäureester der Formel I 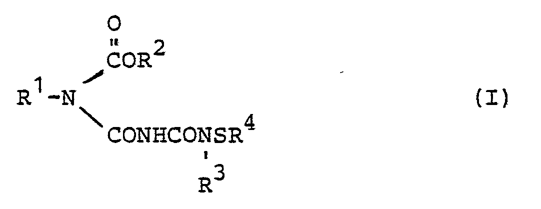 gefunden, in welcher
R und R² gleich oder verschieden sein können und für einen gegebenenfalls substituierten aliphatischen, cyloaliphatischen, aromatischen oder araliphatischen Rest stehen,
_{R}³ für Wasserstoff oder für einen gegebenenfalls substituierten aliphatischen, cycloaliphatischen, araliphatischen oder aromatischen Rest und
_{R}⁴ für einen _{T}rihalogenmethylrest steht.

Man erhält die N-sulfenylierten Biuret-N"-carbonsäureester der Formel (I), wenn man ein Acylisocyanat der Formel II, 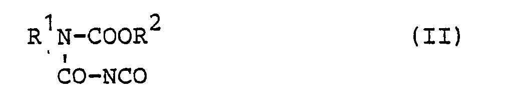 in welcher
R¹ und R² gleich oder verschieden sein können und für einen gegebenenfalls substituierten aliphatischen, cycloaliphatischen, aromatischen oder araliphatischen Rest stehen,
mit einem Sulfenamid der Formel III, 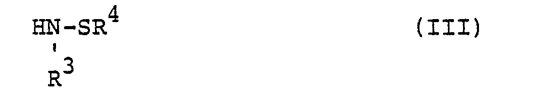 in welcher
_{R}³ für Wasserstoff oder für einen gegebenenfalls substituierten aliphatischen, cycloaliphatischen, araliphatischen oder aromatischen Rest und
R⁴ für einen _{T}rihalogenmethylrest steht,

gegebenenfalls in Gegenwart eines Verdünnungsmittels umsetzt.

Die neuen N-sulfenylierten Biuret-N"-carbonsäureester der Formel I weisen starke fungizide und bakterizide Eigenschaften auf. Zum Teil zeigen sie auch eine akarizide Wirkung und eine Wirkung gegen Hygiene- und Vorratsschädlinge. überraschenderweise zeigen die erfindungsgemäßen Verbindungen der Formel I eine erheblich höhere fungizide Wirkung als die bekannten Verbindungen gleicher Wirkungsrichtung, ebenso ist die sehr gute bakterizide Wirkung im Reis hervorzuheben, die bei den erfindungsgemäßen Verbindungen den aus dem Stand der Technik bekannten Verbindungen überlegen ist. Sie stellen somit eine Bereicherung der Technik dar.

Von den erfindungsgemäßen N-sulfenylierten Biuret-N"- carbonsäuree.stern der Formel I sind bevorzugt diejenigen, bei denen
R¹ und R² gleich oder verschieden sind und für gegebenenfalls substituiertes C₁-C₁₀ Alkyl, C₂-C₁₀ Alkenyl oder C₂-C₁₀ Alkinyl, für gegebenenfalls substituiertes C₅-C₁₀ Cycloalkyl, für gegebenenfalls substituiertes Aralkyl mit C₆-C₁₀ im Arylteil und C₁-C₄ im Alkylteil, wobei der Alkylteil geradkettig oder verzweigt sein kann oder für gegebenenfalls substituiertes C6-C10 Aryl steht,
_{R}³ für Wasserstoff, gegebenenfalls substituiertes C₁-C₁₀ Alkyl, C₂-C₁₀ Alkenyl oder C₂-C₁₀ Alkinyl, für gegebenenfalls substituiertes C₅-C₁₀ Cycloalkyl, für gegebenenfalls substituiertes Aralkyl mit C₆-C₁₀ im Arylteil und C₁-C₄ im Alkylteil, wobei der Alkylteil geradkettig oder verzweigt sein kann oder für gegebenenfalls substituiertes C₆-C₁₀ Aryl steht und
R⁴ für einen Trihalogenmethylrest steht.

Von den erfindungsgemäßen N-sulfenylierten Biuret-N"-car- bonsäureestern der Formel I sind besonders bevorzugt diejenigen, bei denen
_{R}¹, _{R}² und _{R}³ gleich oder verschieden sind und für einen gegebenenfalls durch C₁₋₄ Alkoxy, C₁-C₄ Alkylthio und/ oder Halogen, bevorzugt Fluor, Chlor, Brom und Jod, insbesondere Fluor und Chlor, substituiertes C₁-C_{10'} insbesondere C₁-C₆ Alkyl oder C₂-C₁₀, insbesondere C₃-C₅ Alkenyl oder C₂-C₁₀, insbesondere C₃-C₅ Alkinyl, steht, ferner für gegebenenfalls durch C₁-C₆ Alkyl substituiertes C₅-C_{10'} insbesondere C₅ oder C₆ Cycloalkyl, für gegebenenfalls im Arylteil durch Halogen, bevorzugt Fluor, Chlor, Brom und Jod, insbesondere Fluor und Chlor, Nitro, C₁-C₆ Alkyl, Cyano und/oder Trifluormethyl substituiertes Aralkyl mit C₆-C₁₀ im Arylteil und C₁-C₄, vorzugsweise C₁ oder C₂ im Alkylteil steht, wobei der Alkylteil geradkettig oder verzweigt sein kann, für einen gegebenenfalls durch Halogen, bevorzugt Fluor, Chlor, Brom und Jod, insbesondere Fluor und Chlor, Nitro, Cyano, C₁-C₆, insbesondere C₁-C₄ Alkyl, C₁-C₆, insbesondere C₁-C₄ Alkoxy, Trifluormethyl und/oder Dimethyldihydrofuranyl, substituiertes C₆-C₁₀ Aryl steht,
_{R}³ außerdem für Wasserstoff steht und
_{R}⁴ für einen Trihalogenmethylrest, bevorzugt Trichlormethyl oder Fluordichlormethyl steht.

Ganz besonders bevorzugt sind diejenigen Verbindungen der Formel I, in denen
_{R}¹, _{R}² und _{R}³ gleich oder verschieden sind und für einen gegebenenfalls durch Methoxy, Ethoxy, n- und i-Propoxy, Methylthio, Ethylthio, n- und i-Propylthio, Fluor und/oder Chlor substituiertes C₁-C₆ Alkyl oder C₃-C₅ Alkenyl oder C₃-C₅ Alkinyl steht, weiterhin für gegebenenfalls durch Methyl, Ethyl, n- und i-Propyl, n-, s-, i- und t-Butyl substituiertes C₅ oder C₆ Cycloalkyl, für gegebenenfalls im Arylteil durch Fluor, Chlor, Nitro, Cyano, Methyl, Ethyl, n- und i-Proppyl, n-, s-, i- und t-Butyl und Trifluormethyl substituiertes Arylalkyl, vorzugsweise Benzyl und Phenylethyl steht, ferner für gegebenenfalls durch Fluor, Chlor, Nitro, Methyl, Ethyl, n- und i-Propyl, n-, i-, s- und t-Butyl, Methoxy, Ethoxy, n- und i-Propoxy, Trifluormethyl und/oder Dimethyldihydrofuranyl substituiertes C₆-C₁₀ Aryl, insbesondere Phenyl und Naphthyl und
R³ außerdem für Wasserstoff und
_{R}⁴ für Trichlormethyl und Fluordichlormethyl steht.

Die angeführten Reste können ein- oder mehrfach, gleich oder verschieden substituiert sein.

Neben den in den Herstellungsbeispielen beschriebenen Verbindungen der Formel (I) seien im einzelnen die folgenden Verbindungen genannt:

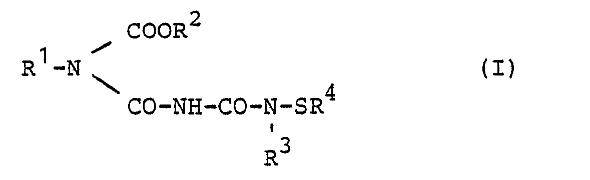

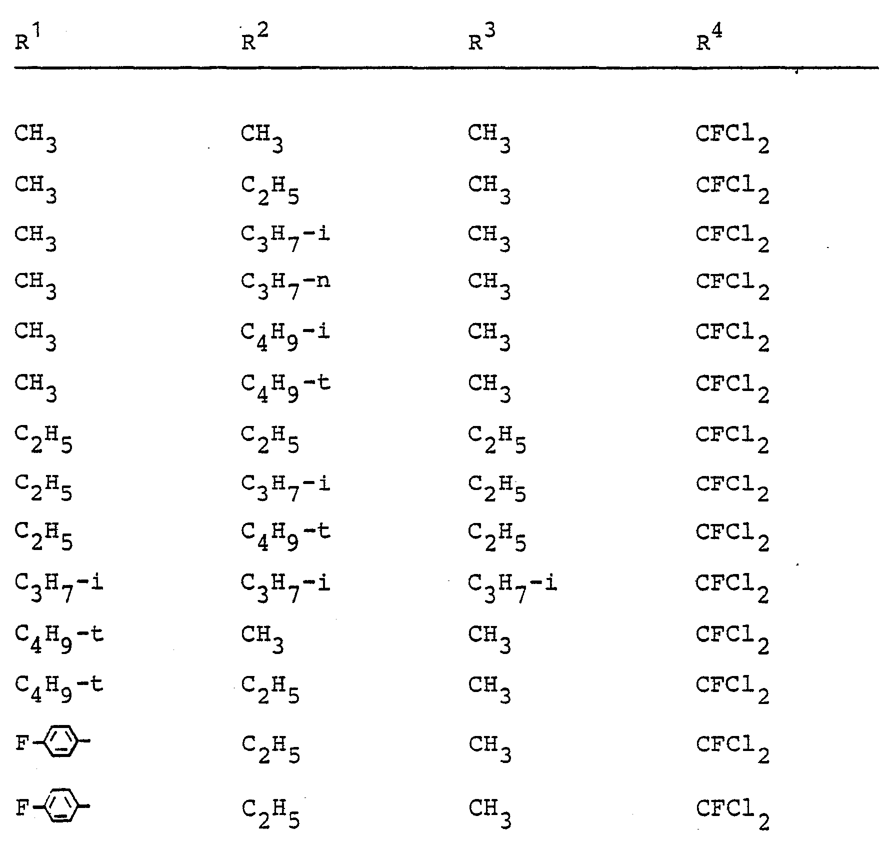

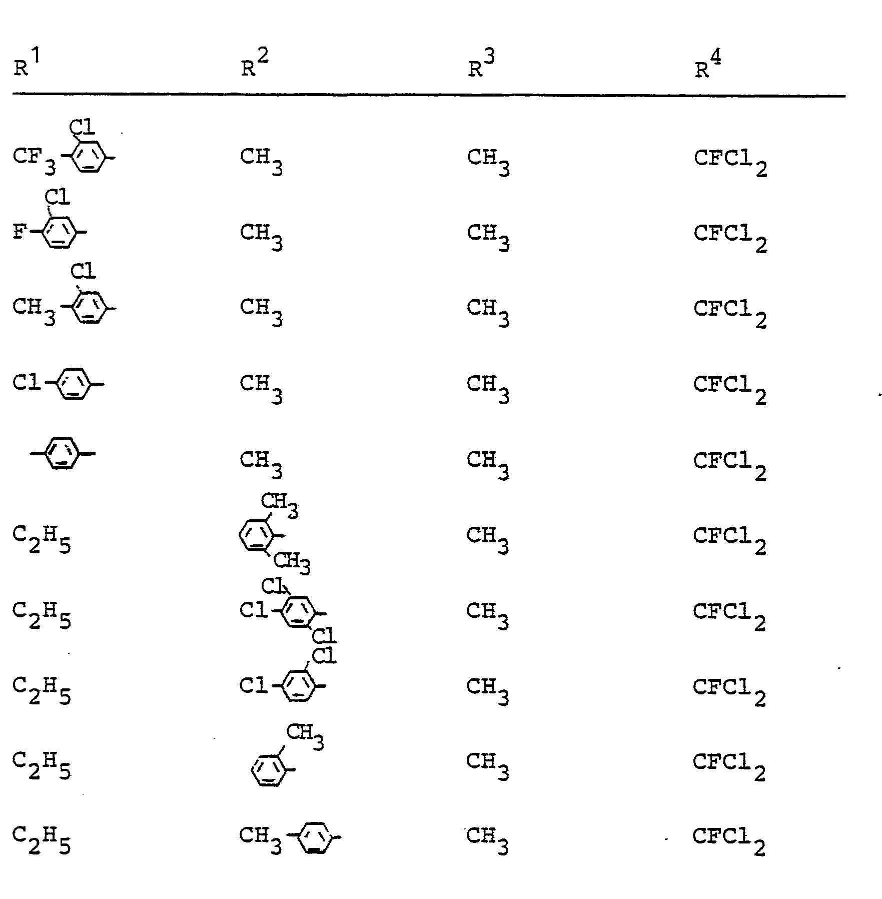

Verwendet man z.B. zur Herstellung der erfindungsgemäßen Verbindungen der Formel (I) N-Methyl-N-phenoxycarbonyl-N-carbonylisocyanat und das N-Ethyl-fluordichlormethansulfenamid als Ausgangskomponenten, so kann der Reaktionsverlauf durch das nachfolgende Reaktionsschema wiedergegeben werden:

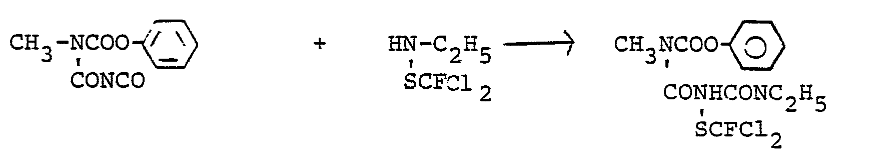

Die als Ausgangsstoffe für die Herstellung der erfindungsgemäßen Verbindungen der Formel (I) benötigten Acylisocyanate sind durch die allgemeine Formel (II) definiert. In dieser Formel haben R¹ und R² die vorher genannten Bedeutungen.

Die Verbindungen der Formel II, in denen _{R}¹ und _{R}² für Alkyl steht, sind bekannt und können nach literaturbekannten Verfahren hergestellt werden (Synthesis 1980, 112). Außerdem können die Ausgangsverbindungen der Formel II nach einem nicht zum Stand der Technik gehörenden Verfahren hergestellt werden, indem man N-substituierte Carbamidsäureester der Formel IV 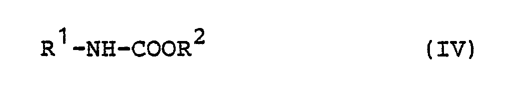 in welcher
_{R}¹ und R² die oben angegeben Bedeutung haben, mit Chlorcarbonylisocyanat der Formel V 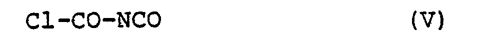 in einem Verdünnungsmittel bei Temperaturen zwischen 50 und 200°C umsetzt (vergleiche auch Herstellungsbeispiele).

Das zu verwendende Chlorcarbonylisocyanat der Formel V ist bekannt, ebenso die N-substituierten Carbamidsäureester der Formel IV.

Die erfindungsgemäß verwendbaren Ausgangsverbindungen der Formel (III) sind bekannt oder können nach bekannten Verfahren hergestellt werden (FR-PS 1 339 765 bzw. Chem. Abstr. 60, 5519 (1964)). Man erhält sie, wenn man Trihalogenmethansulfenchlorid mit einem primären Amin beispielsweise in Toluol als Lösungsmittel im Temperaturbereich zwischen 0 und 30°C zur Umsetzung bringt (vgl. auch Herstellungsbeispiele).

Die erfindungsgemäße Reaktion wird vorzugsweise in Anwesenheit eines Verdünnungsmittels durchgeführt. Als Verdünnungsmittel können alle inerten Lösungsmittel verwendet werden wie Kohlenwasserstoffe z.B. Toluol, chlorierte Kohlenwasserstoffe z.B. Chlorbenzol oder Ether, wie z.B. Dioxan.

Die Reaktionstemperaturen können in einem größeren Bereich variiert werden. Im allgemeinen arbeitet man zwischen 0 und 150°C, vorzugsweise bei 20° bis 120°C.

Die Umsetzung wird vorzugsweise bei Normaldruck durchgeführt.

Bei der Durchführung der Umsetzung geht man vorzugsweise so vor, daß das Acylisocyanat der Formel II gelöst in einem der angegebenen Verdünnungsmittel vorgelegt wird und das Sulfenamid der Formel III bei Raumtemperatur zugetropft wird. Die Reaktionstemperatur steigt dabei an. Die Reaktionslösung wird durch Destillation im Vakuum vom Verdünnungsmittel befreit. Der Rückstand kann aus einem organischen Lösungsmittel umkristallisiert werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Thizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Pyricularia oryzae, dem Erreger der Brusone Krankheit, zur Bekämpfung von Leptosphaeria nodorum im Weizen und von Xanthomonas oryzae im Reis eingesetzt werden. Außerdem weisen die Verbindungen eine Wirkung gegen Phytophthora und gegen Botrytis auf.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kiesesäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor,-Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischungen mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen,Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich. Herstellungsbeispiele:

### Beispiel 1

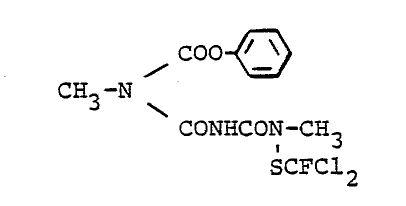

14 g (0,063 Mol) N-Methyl-N-(isocyanatocarbonyl)-0-phenylcarbamat werden in 50 ml Dioxan gelöst und bei Raumtemperatur tropfenweise mit 105 g (0,07 Mol) Fluordichlormethansulfenyl-N-methylamid versetzt. Hierbei steigt die Temperatur bis 60°C an. Man engt die Reaktionslösung im Vakuum ein und kristallisiert den Rückstand aus Methanol um. Man erhält 18 g (78 % der Theorie) N"-Methyl-N"-phenoxycarbonyl-N-methyl-N-fluordichlor- methylthio-biuret vom Schmelzpunkt 119 - 120°C.

In analoger Weise erhält man die in der nachstehenden Tabelle aufgeführten Verbindungen der Formel

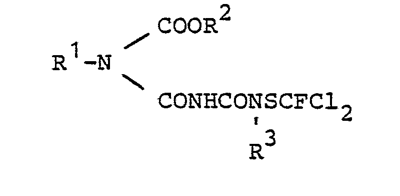

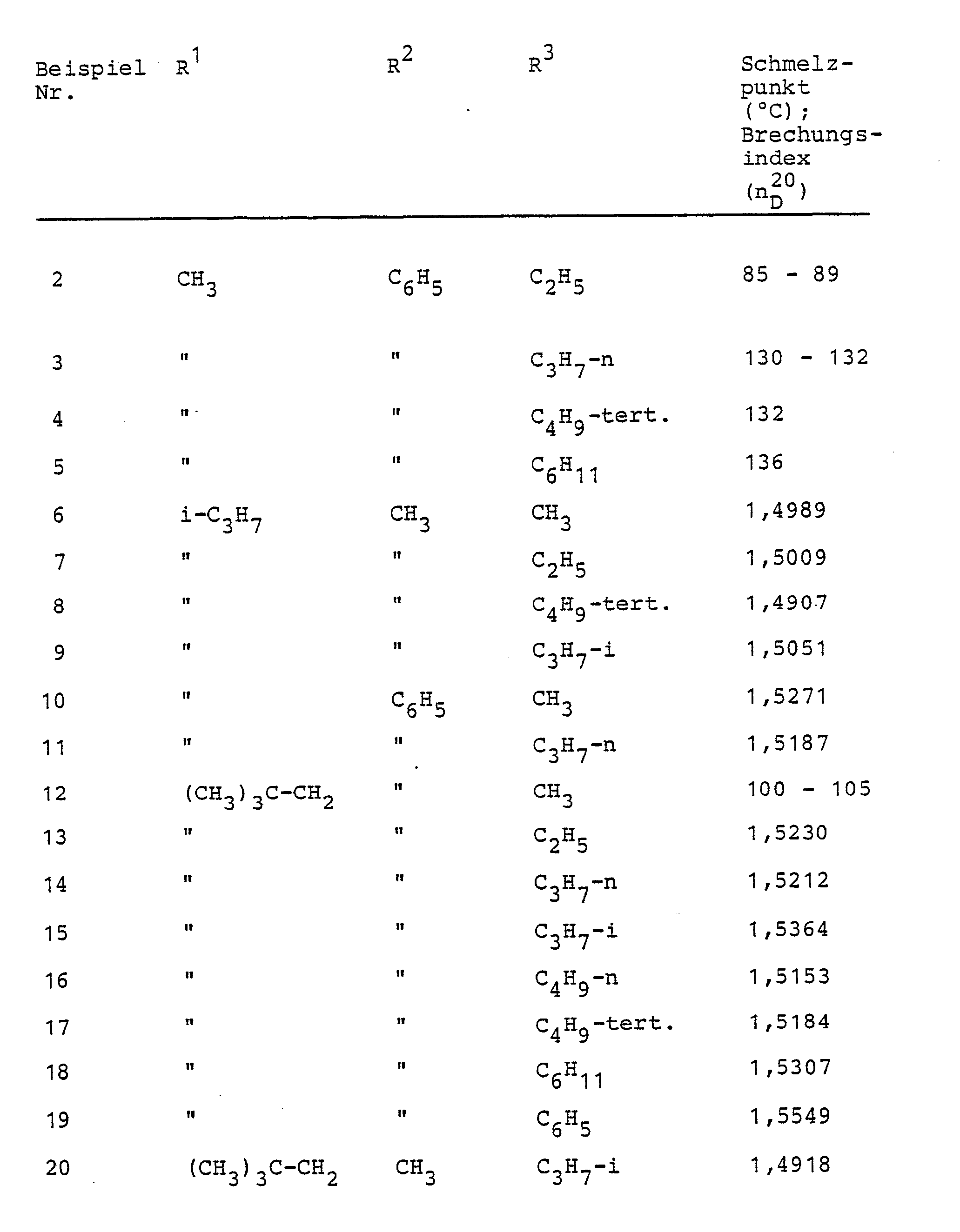

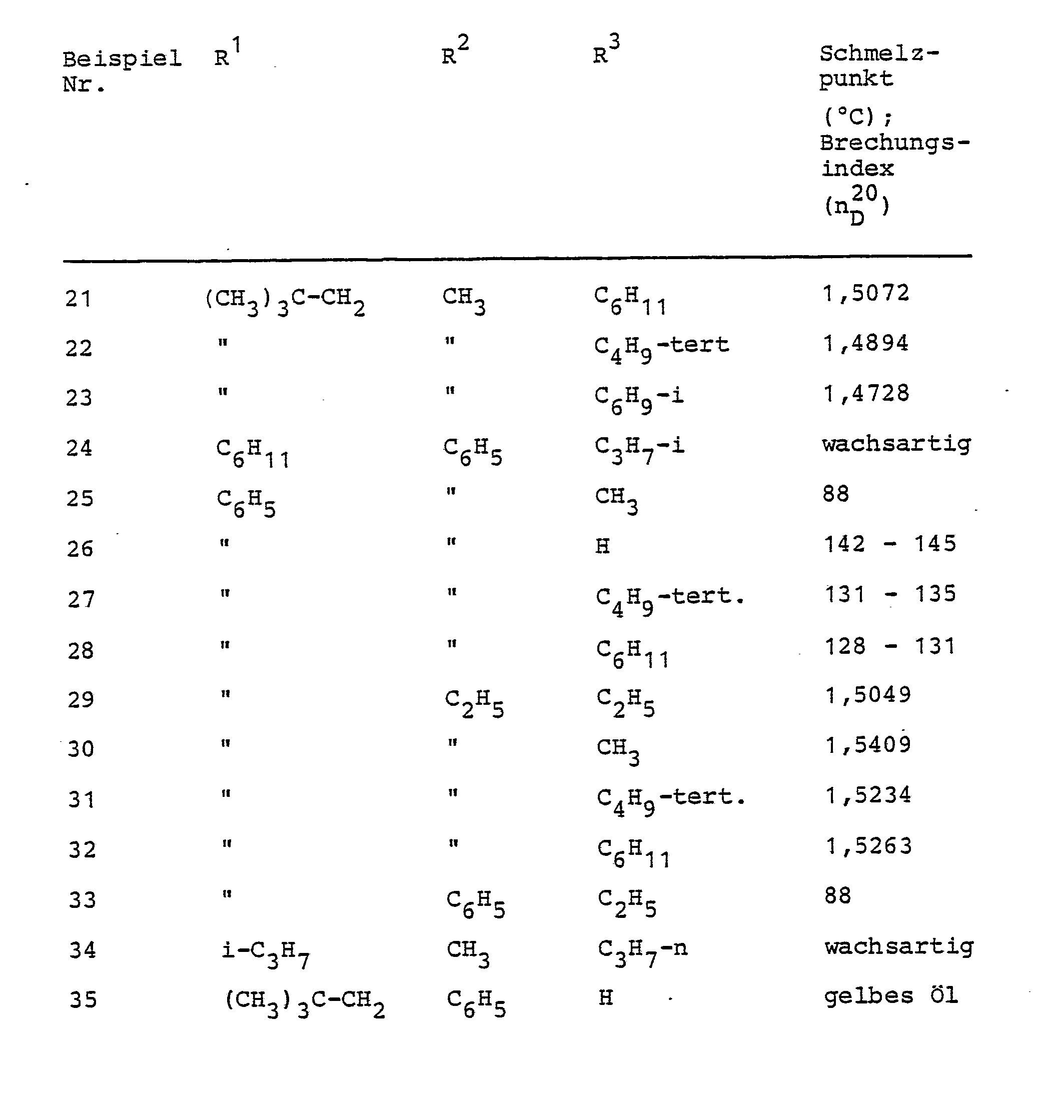

Herstellung der Ausgangsprodukte der Formel II

### Beispiel A

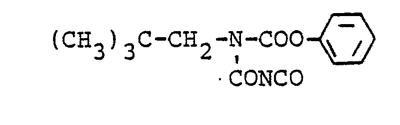

62 g (0,3 Mol) N-Neopentylcarbamidsäure-O-phenylester werden in 200 ml trockenem Chlorbenzol gelöst und zu einer Lösung von 35 g (0,33 Mol) Chlorcarbonylisocyanat in 70 ml trockenem Chlorbenzol getropft. Bei Raumtemperatur findet keine merkliche Reaktion statt. Beim Erhitzen der Reaktionslösung entwickelt sich ab etwa 95°C fortlaufend Chlorwasserstoff. Nach etwa 1,5 - 2 Stunden ist diese Gasentwicklung mit Erreichen der Siedetemperatur des Chlorbenzols beendet. Man destilliert das Lösungsmittel im Vakuum ab. Der Rückstand liefert durch Hochvakuumdestillation 68 g (88 % der Theorie) N-Neopentyl-N-carbonylisocyanato-carbaminsäure-O-phenylester mit dem Siedepunkt von 111 - 112°C/0,1 Torr.

In analoger Weise erhält man folgende Produkte der Formel IV

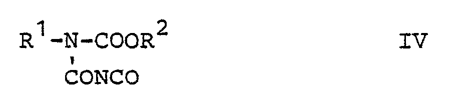

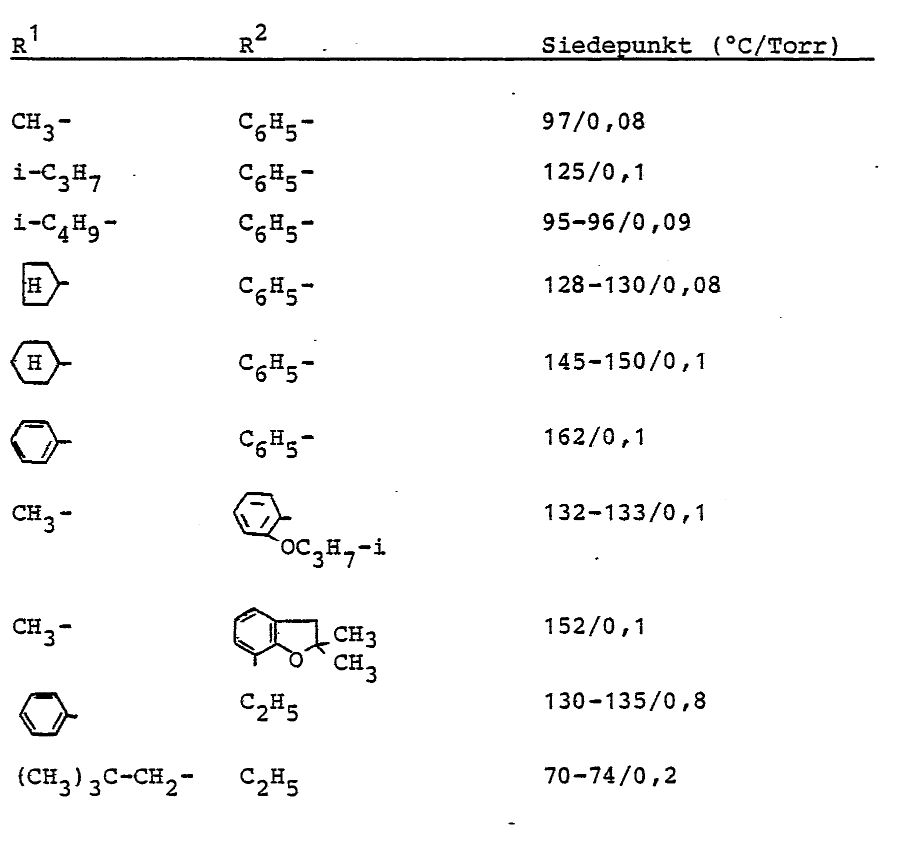

### Herstellung der Vorprodukte der Formel III

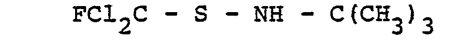

73 g (1 Mol) tert.-Butylamin werden in 300 ml Toluol gelöst und unter Kühlung bei 20 bis 30°C tropfenweise mit 85 g (0,5 Mol) Fluordichlormethan-sulfenylchlorid versetzt. Man schüttelt die Lösung mit Wasser aus, trocknet über Natriumsulfat, engt die Toluol-Lösung im Vakuum ein und destilliert den Rückstand. Man erhält 80 g (77 % der Theorie) Fluordichlormethan-sulfenyl-N-(t-butyl)-amid vom Siedepunkt 60 - 65°C/13 Torr.

In den nachfolgenden Beispielen werden die nachstehend angegebenen Verbindungen als Vergleichssubstanzen eingesetzt:

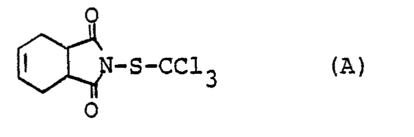

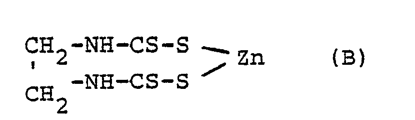

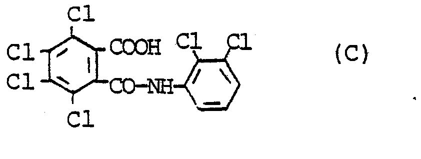

### Beispiel A

### Leptosphaeria nodorum-Test (Weizen) / protektiv

Lösungsmittel: 100 Gewichtsteile Dimethylformamid
Emulgator: 0,25 Gewichsteile Alkylaryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber der aus dem Stand der Technik bekannten Verbindung A zeigen bei diesem Test z.B. die Verbindungen der Herstellungsbeispiele 1, 6, 20, 22, 33, 2, 3, 10, 11 und 9.

### Beispiel B

### Pyricularia-Test (Reis) / protektiv

Lösungsmittel: 12,5 Gewichtsteile Aceton
Emulgator: 0,3 Gewichtsteile Alkylarylpolyglykol-ether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur _{T}ropfnässen. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in.der Wirksamkeit gegenüber der aus dem Stand der Technik bekannten Verbindung B zeigen bei diesem Test z.B. die Verbindungen der Herstellungsbeispiele: 12, 25, 13, 7, 8, 34 und 33.

### Beispiel C

### Xanthomonas oryzae-Test / Bakteriose / Reis protektiv

Lösungsmittel: 24,25 Gewichtsteile Aceton
Emulgator: 0,75 Gewichtsteile Alkylarylpolyglykol-ether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach dem Antrocknen des Spritzbelages werden die Pflanzen mit einer wäßrigen Suspension von Xanthomonas oryzae durch Stichverletzung der Blätter inokuliert. Nach einer 48-stündigen Inkubation bei 100 % rel. Luftfeuchtigkeit verbleiben die Pflanzen 10 Tage bis zur Auswertung in einem Gewächshaus bei 24 bis 26°C und 70 bis 80 % rel. Luftfeuchtigkeit.

Eine deutliche Überlegenheit in der Wirksamkeit gegenüber der aus dem Stand der Technik bekannten Verbindung C zeigen bei diesem Test z.B. die Verbindungen der Herstellungsbeispiele: 35, 12, 13 und 14.

## Patentansprüche

1. N-Sulfenylierte Biuret-N"-carbonsäureester der Formel I 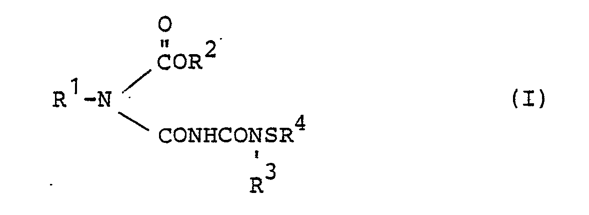 in welcher
_{R}¹ und R² gleich oder verschieden sein können und für einen gegebenenfalls substituierten aliphatischen,cycloaliphatischen, aromatischen oder araliphatischen Rest stehen,
_{R}³ für Wasserstoff oder für einen gegebenenfalls substituierten aliphatischen, cycloaliphatischen, araliphatischen oder aromatischen Rest und
_{R}⁴ für einen Trihalogenmethylrest steht.

2. Verfahren zur Herstellung von N-sulfenylierten Biuret-N"-carbonsäureestern der Formel 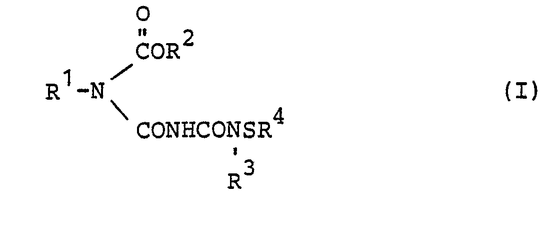 in welcher
R¹ und R² gleich oder verschieden sein können und für einen gegebenenfalls substituierten aliphatischen, cycloaliphatischen, aromatischen oder araliphatischen Rest stehen,
_{R}³ für Wasserstoff oder für einen gegebenenfalls substituierten aliphatischen, cycloaliphatischen, araliphatischen oder aromatischen Rest und
_{R}⁴ für einen Trihalogenmethylrest steht,
dadurch gekennzeichnet, daß man ein Acylisocyanat der Formel II, 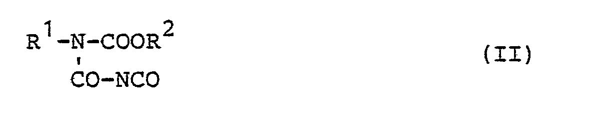 in welcher
_{R}¹ und R² die oben genannte Bedeutung haben,
mit einem Sulfenamid der Formel III, 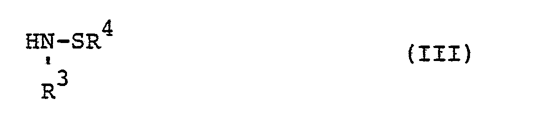 in welcher
_{R}³ und _{R}⁴ die oben angegebene Bedeutung haben,
gegebenenfalls in Gegenwart eines Verdünnungsmittels umsetzt.

3. Schädlingsbekämpfungsmittel, gekennzeichnet durch einen Gehalt an mindestens einem N-sulfenylierten Biuret-N"-carbonsäureester der Formel (I) gemäß Anspruch 1 und 2.

4. Verwendung von N-sulfenylierten Biuret-N"-carbon- säureestern der Formel (I) zur Bekämpfung von Schädlingen.

5. Verfahren zur Bekämpfung von Schädlingen, dadurch gekennzeichnet, daß man N-sulfenylierte Biuret-N"- carbonsäureester der Formel (I) auf Schädlinge und/ oder ihren Lebensraum einwirken läßt.

6. Verfahren zur Herstellung von Schädlingsbekämpfungsmitteln, dadurch gekennzeichnet, daß man N-sulfenylierte Biuret-N"-carbonsäureester der Formel (I) mit Streckmitteln und/oder oberflächenaktiven Mitteln vermischt.
